# EUROPEAN PATENT SPECIFICATION

(11) **EP 3 481 811 B1**
(45) Date of publication and mention of the grant of the patent: **25.05.2022**
(21) Application number: 17739701.5
(22) Date of filing: 07.07.2017
(51) Int. Cl.: C07D 295/06, C07C 275/26

(54) **INDUSTRIAL PROCESS FOR THE PREPARATION OF CARIPRAZINE**
INDUSTRIELLES VERFAHREN ZUR HERSTELLUNG VON CARIPRAZIN
PROCESSUS INDUSTRIEL POUR LA PRÉPARATION DU CARIPRAZINE

(30) Priority: 08.07.2016 HU 1600420; 09.05.2017 HU 1700197
(43) Date of publication of application: 15.05.2019
(60) Divisional application: 22165881.8
(73) Proprietor: Richter Gedeon Nyrt., 1103 Budapest (HU)
(72) Inventor: NEU, József, H-1133 Budapest (HU); GARADNAY, Sándor, 1037 Budapest (HU); SZABÓ, Tamás, 2510 Dorog (HU)
(74) Representative: Hoffmann Eitle
(86) International application number: PCT/IB2017/054094
(87) International publication number: WO 2018/007986

(56) References cited:
- WO-A1-03/029233
- WO-A1-2005/012266
- WO-A1-2010/070368
- WO-A1-2014/031162
- CN-A- 105 218 484
- CN-A- 105 330 616
- XIAOWEN CHEN ET AL: "A New and Practical Synthesis of Cariprazine through the Facile Construction of 2-[trans-4-(3,3-Dimethylureido)cyclohexyl] acetic Acid", SYNTHESIS, vol. 48, no. 18, 23 June 2016 (2016-06-23) , pages 3120-3126, XP055414612, STUTTGART, DE. ISSN: 0039-7881, DOI: 10.1055/s-0035-1561865

## Description

### Field of the Invention

The present invention provides an industrial process for the preparation of trans-N-{4-[2-[4-(2,3-dichlorophenyl)-piperazin-1-yl]-ethyl]-cyclohexyl}-N',N'-dirriethylurea having the formula (1), commonly known as cariprazine.

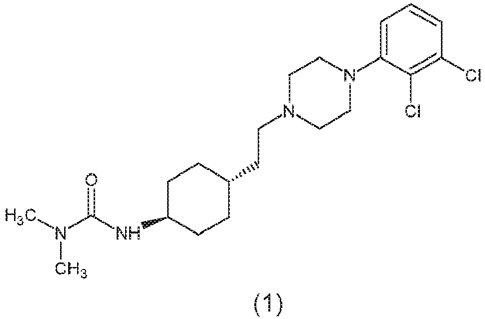

### Technical Field

The active agent dopamine D3/D2 receptor antagonist and its synthesis method was disclosed in the international patent application WO 2005/012266A1 first time. According to this process the base released from trans-N-{4-[2-[4-(2,3-dichlorophenyl)-piperazin-1-yl]-ethyl]-cyciohexyl}-N',N'-dirriethylurea trihydrochloride is reacted with triphosgene. The targeted compound is received by crystallization of the product in methanol. Among others the disadvantages of this process include use of triphosgene for the development of isocyanate. Triphosgene, the compound itself is extremely toxic chemical compound and its use requires special arrangements under industrial conditions.

Trans-4-{2-[4-(2,3-dichlorophenyl)piperazin-1-yl]-ethyl}-cyclohexyl-amine trihydrochloride, the starting material of synthesis in accordance with the international patent application WO2005/012266A1, is produced by a well-known method disclosed in the international patent application WO2003/029233A1. Accordingly, 1-(2,3-dichlorophenyl)piperazine is coupled with trans-Boc-2-(4-aminocyclohexyl)acetaldehyde in a step of reductive amination, then from the intermediate compound the tert-butyloxycarbonyl protective group is cleaved with ethyl acetate in hydrochloric acid. Due to the chemical reaction carried out in dichloroethane realization of industrial scale process is hampered severely.

Trans-Boc-2-(4-aminocyclohexyl) acetic acid ethyl ester, the starting material for production of the aldehyde reagent is produced in the manner described in international patent application WO2010/070368A1. Accordingly, 4-nitrophenyl acetic acid is hydrogenated in the presence of palladium on carbon at 0,1-0,6 bar overpressure on temperature between 40°C and 50°C, which is followed by "one pot" esterification to form trans-Boc-2-(4-aminocyclohexyl) acetic acid ethyl ester. Then from this reaction mixture trans-Boc-2-(4-aminocyclohexyl)acetaldehyde is obtained by partial reduction method described in the publication of Journal of Medicinal Chemistry, 2000, vol. 43, #9 p. 1878-1885. The main disadvantage of this reduction is that due to suppression of the excessive hydrogenation DIBALH reagent has to be added to the reaction mixture on -78°C, this temperature represents a difficult challenge to be met in most industrial plants.

The other reaction partner used in the coupling reaction mentioned above, the 1-(2,3-dichlorophenyl)-piperazine can be produced from piperazine and 1-bromo-2,3-dichlorobenzene with Buchwald reaction method according to the publication of Bioorganic and Medicinal Chemistry, 2002, vol. 10, # 12 p. 4023-4027.

The 1-(2,3-dichlorophenyl)-piperazine is N-alkylated with 2-{trans-4-[(tert-butoxycarbonyl)amino]cyclohexyl}ethyl methanesulfonat in the manner disclosed in international patent application WO2010/070369. 2-{Trans-4-[(tert-butoxycarbonyl)amino]cyclohexyl}ethyl methanesulfonat is obtained from trans-Boc-2-(4-aminocyclohexyl)acetic acid ethylester by reduction which is followed by reaction with methanesulfonyl chloride. The disadvantage of the process is that methanesulfonate derivatives are remaining in the reaction mixture, which are considered potential genotoxic contamination, and detection of that can be difficult and the extent of this contamination should be kept at ppm level. Relevant information can be found for example on ACS Publications website at DOI: 10.1021/cr300095f. Chemical Reviews Gyorgy Szekely et al. Genotoxic Impurities in Pharmaceutical Manufacturing: Sources, Regulations, and Mitigation.

According to international patent application WO2015/056164 the protected derivative of trans-4-aminocyclohexyl acetic acid is reduced to alcohol with diisobutylaluminum hydride in tetrahydrofuran solution, which is followed by direct coupling of the obtained alcohol with 1-(2,3-dichlorophenyl)-piperazine. In the coupling reaction tri-ruthenium dodecacarbonyl complex formed with Xantophos is used. The downside of the procedure is the application of a costly catalyst and a ligand, in addition to that the cost-efficiency is reduced by chromatographic purification of the coupled product significantly.

A Chinese patent application having publication number of CN105330616A describes a method where cariprazine is produced with a process starting from ketone compound, namely from 4-(2-ethyl-hydroxyethyl)cyclohexanone. The first step in the production process is a Mitsunobu coupling reaction, to which diethyl azodicarboxylate is used. The reagent is known to be explosive, so this process cannot be scaled-up and it can be carried out in the industry with special preparations only. Afterwards, the resulting condensation intermediate can be converted into the corresponding cyclohexyl amine with hydroxylamine hydrochloride in the presence of benzyl amine or ammonia reducing reagent. Finally, the target compound is obtained by acylation with dimethyl-carbamoyl chloride. Moreover, the used starting compound 4-(2-hydroxyethyl) cyclohexanone is difficult to access, its production is a complicated process. During its production reagents and special protecting groups demanding unusual preparations can cause certain difficulties, which decreases the overall production efficiency.

According to the patent application US2001009912A1 in the first step 4-hydroxyphenyl-acetic acid ethyl ester is hydrogenated on 160°C at 200 bar pressure for 72 hours, then the so obtained (4-hydroxycyclohexyl) acetic acid ethyl ester is oxidized with the extremely expensive Dess-Martin's periodate in order to produce (4-oxocyclohexyl) acetic acid ethylester.

According to the international patent application WO2006/44524A1 4-hydroxyphenylacetic acid ester is reduced with expensive rhodium catalyst. The starting material of the synthesis disclosed in Chinese patent application CN105330616A can be produced from ethyl-(4-oxo-cyclohexyl) acetate by introduction of different protecting groups only.

Taking into account all the listed solutions, we have set the goal of developing a new alternative cariprazine synthesis that is industrially feasible. The method can be accomplished through the synthesis of new intermediate compounds which have not been synthesized so far.

### Summary of the invention

The invention provides a process for the preparation of cariprazine 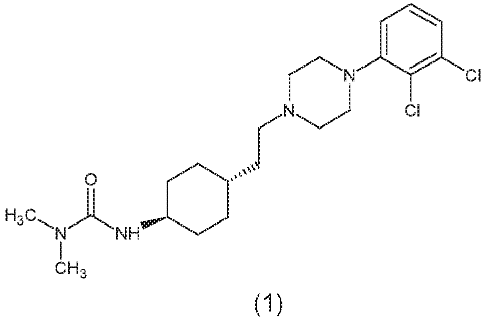 wherein
a) (trans-4-amino-cyclohexyl) acetic acid ethyl ester hydrochloride is converted to trans-4-aminocyclohexyl)-acetic acid or its hydrochloride by hydrolysis,
b) from the obtained product with addition of a dicarbamoyl derivative selected from a halide, imidazolide, anhydride or active ester as a suitable reagent in presence of alkaline reagent (trans-4-{[(dimethylamino)carbonyl]amino} cyclohexyl) acetic acid is formed,
c) then the obtained compound is linked to 1-(2,3-dichlorophenyl)-piperazine in the presence of carboxylic acid activating coupling reagent, and so 1,1-dimethyl-3-[trans-4-(2-oxo-2-(4-(2,3-dichlorophenyl)piperazin-1-yl-ethyl)cyclohexyl] urea is formed.
d) which is converted to cariprazine borane adduct of formula (2), 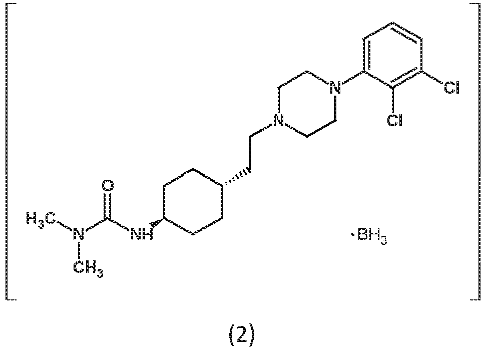 in the presence of a borohydride compound, borane or a borane complex as a reducing agent,
e) and final product itself is eliminated directly or is obtained from its salt.

Intermediate compounds formed and/or used in the process of the present invention include 1,1-dimethyl-3-[trans-4-(2-oxo-2-(4-(2,3-dichlorophenyl)piperazin-1-ylethyl)cyclohexyl] urea and cariprazine borane adduct.

### Detailed description of the invention

(Trans-4-aminocyclohexyl) acetic acid ethyl ester hydrochloride, the starting compound of the new process for preparation of cariprazine according to the present invention, is produced by the method disclosed in the international patent application WO2010/70368A1. This compound is converted into (trans-4-aminocyclohexyl) acetic acid or its hydrochloride by hydrolysis, from which by adding a suitable dimethyl-carbamoyl derivative in presence of alkaline reagent (trans-4 {[(dimethylamino)carbonyl]aminolcyclohexyl)-acetic acid is formed.

In order to form carboxylic acid (trans-4-aminocyclohexyl)-acetic acid ethyl ester hydrochloride is hydrolyzed under acidic or alkaline conditions by using reagents generally known from the literature, e. g. form Wuts, Peter G. M.: Greene's protective groups in organic synthesis - 4th edition 543-544.

During the preparation of (trans-4{[(dimethylamino)carbonyl]amino}cyciohexyl)-acetic acid as a new chemical compound the amine group of (trans-4-aminocyclohexyl) acetic acid is reacted with a suitable halide, imidazolide, anhydride, or active ester of the dimethyl-carbamoyl acid, for example it is reacted with dimethyl-carbamoyl chloride in presence of an alkaline reagents such as sodium bicarbonate.

The obtained compound is linked to 1-(2,3-dichlorophenyl)-piperazine and so 1,1-dimethyl-3-[*trans*-4-(2-oxo-2-(4-(2,3-dichlorophenyl)piperazin-1-yl-ethyl)cyclohexyl] urea is provided. Only that kind of coupling reagents are suitable for the purpose of this chemical reaction, which are able to activate carboxylic acid and facilitate acylation of the secondary amino group of 1-(2,3-dichlorophenyl)-piperazine with trans-4-{[(dimethylamino)carbonyl]amino} cyclohexyl) acetic acid. These reagents, which can be also dehydrating agents, are reagents able to form an acid chloride from carboxylic acid such as thionyl chloride; agents capable to form reactive acyl-imidazolyl derivatives such as carbonyldiimidazole, carbodiimide; compounds such as N,N'-dicyclohexyl-carbodiimide and N,N'-diisopropyl-carbodiimide, that kind of benztriazole derivatives such as HATU, HBTU, HOBt or azabenzotriazoles such as PyAOP, PyBOP, HOAt are also can be used, and in addition to that boric acids, boronic acids, or the reagents that are well known by skilled persons in the art from chapters 16-72, 16-73 and 16-74 of the publication of Michael B. Smith and Jerry March having the title "March's Advanced Organic Chemistry" 6th edition Wiley in 2007 (Print ISBN: 9780471720911 Online ISBN: 9780470084960 DOI: 10.1002/0470084960) can be used as well. Optionally, 1,1-dimethyl-3-[trans-4-(2-oxo-2-(4-(2,3-dichlorophenyl)piperazin-1-ylethyl)cyclohexyl] urea that was formed at the end of the coupling reaction can be further processed in the next step.

The 1,1-dimethyl-3-[trans-4-(2-oxo-2-(4-(2,3-dichlorophenyl)piperazin-1-ylethyl)cyclohexyl] urea in the presence of reducing agent can be converted into the targeted compound. It is reacted with a reducing agent that is able to reduce amide functional group presented in the compound into a suitable amine while the urea functional group in the molecule structure remains unchanged under the conditions of the reaction, and so the targeted compound can be obtained. Suitable reducing agents include a borohydride compound, borane or a borane complex.

In comparison with processes described so far the advantage of the developed process is that utilization of protective group during the synthesis it is not necessary, and so the product manufacturing technology is simplified and the amount of materials used decreases accordingly, and in addition to that the emission of byproducts which are often classified as harmful substances to the environment is lower.

The Boc-trans-2-(4-aminocyclohexyl) acetic acid ethyl ester key intermediate compounds of the manufacturing processes disclosed in the international patent applications WO2003/029233, WO2010/070369 and WO2015/056164 has two protecting groups, namely the ester functional group and the Boc protecting group. For formation or removal of these functional groups separate chemical steps are required which are not necessary for the present invention. There is no need for reduction at low temperature; no solvents that prohibited in the industrial scale production are used; methanesulphonyl chloride is not used, which could form alkyl mesylate with alcohols, particularly methyl mesylate with methanol in the rest of the procedure. Additionally, catalysts (e.g.: Ru₃(CO)₁₂ catalyst) and ligands (e.g.: Xantphos) and method of preparative chromatography which all significantly increase the cost of the process are not used.

According to the process of the present invention during the hydrolysis of trans-4-aminocyclohexyl) acetic acid ethyl ester hydrochloride the starting material can be hydrolyzed both in acidic or basic medium. As a base alkali metal hydroxides, particularly sodium hydroxide can be used. Between 2 and 10 equivalent, optimally 2.5 equivalent amount of sodium hydroxide can be used. When acidic hydrolysis is carried out Brönsted acids, particularly hydrochloric acid is used for hydrolysis reaction. The concentration of the hydrochloric acid solution ranges from 2 mol/dm3 to 12 mol/dm3, optimally it is 6 mol/dm3. For both hydrolysis, water, a water miscible solvent, or a mixture of these are used throughout the reaction. In the case of a basic medium, this is mainly methanol-water, in case of acid hydrolysis water is used.

The term preparation of (trans-4-{[(dimethylamino)carbonyl]amino}cyclohexyl)-acetic acid refers to the reaction in which the (trans-4-aminocyclohexyl)-acetic acid is reacted with the corresponding halide, imidazolide, anhydride or an active ester of dimethylcarbamoyl acid, in the presence of bases, especially with dimethylcarbamoyl chloride. The amount of dimethylcarbamoyl chloride ranges from 1.0 equivalent to 2.0 equivalent, optimally 1.2 equivalent. Bases are referred to as Brönsted bases, mainly alkali metal carbonates and hydrogen carbonates, tertiary amines, optimally sodium bicarbonate. Acylation catalyst refers to pyridine or dimethylamino-pyridine. As reaction medium mainly water is used, but also hydrocarbons, ethers, esters and ketones, as well as their one-phase and two-phase mixtures with each other or with water can be used.

The 1-(2,3-dichlorophenyl)-piperazine compound is acylated with trans-4-{[(dimethylarnino)carbonyl]amino}cyclohexyl) acetic acid. According to a preferred embodiment during the acylation the corresponding acid chloride is formed from carboxylic acid with thionyl chloride. According to another preferred embodiment the reaction is carried out "one pot" with the piperazine derivative, optionally in the presence of a base or acylation catalyst. 1-5 equivalent, optimally 2 equivalent amount of thionyl chloride is required for the formation of the acid chloride. Bases are referred to as Brönsted bases, mainly alkali metal carbonates and hydrogen carbonates, tertiary amines, preferably sodium bicarbonate or triethylamin. Acylation catalyst refers to pyridine or dimethylamino-pyridine. As reaction medium hydrocarbons, ethers, esters and ketones, as well as their one-phase and two-phase mixtures with each other or with water are used, but mainly toluene, acetone, dichloromethane, tetrahydrofuran can be used.

The acylation reaction step can be carried out also through acid-imidazolide. In this case, (trans-4-{[(dimethylamino)carbonyl]amino}cyclohexyl) acetic acid is dissolved in an appropriate solvent and carbonyl diimidazole is added to the solution. After the formation of the active intermediate 1-(2,3-dichlorophenyl)-piperazine compound is added to the reaction mixture. Finally, from the reaction mixture the desired 1,1-dimethyl-3-[trans-4-(2-oxo-2-(4-(2,3-dichlorophenyl)piperazin-1-ylethyl)cyclohexyl] urea is eliminated by crystallization. 1-5 equivalent, optimally 1-1.5 equivalent amount of carbonyl diimidazole may be used. As reaction medium hydrocarbons, ethers, esters and ketones, as well as their one-phase and two-phase mixtures, but mainly toluene, acetone, dichloromethane, tetrahydrofuran can be used. The reaction is carried out within the liquid range of the solvent, but optimally at a temperature between 20 and 25 °C.

The obtained 1,1-dimethyl-3-[trans-4-(2-oxo-2-(4-(2,3-dichlorophenyl)piperazin-1-yl-ethyl) cyclohexyl] urea is reduced to Carprazine of formula (1). The reduction can be carried out with various types of borohydride compounds, among which the "alumina borohydride" produced in situ from sodium borohydride and aluminum chloride, have been found to be advantageous. In addition, several borane complexes reduce the acid amide to the corresponding amine, selectively. Borane complexes include complexes of borane ethers, optimally their complexes are formed with ether-type solvents. Borane complex refers to the complexes of boranes formed with Lewis bases, such as complexes formed with ethers, thioethers, amines, preferably complex formed with tetrahydrofuran.

Due to the difficult handling of borane, it is advantageously "in situ" formed with sodium borohydride or boron trifluoride etherate, iodine or Brönsted acids, preferably it is formed with boron trifluoride etherate. As a medium of reduction, ether type solvents are used, preferably the solvent may be tetrahydrofuran.

Surprisingly, we have found that after reduction of 1,1-dimethyl-3-[trans-4-(2-oxo-2-(4-(2,3-dichlorophenyl)piperazin-1-yl-ethyl)cyclohexyl] urea with borane reagents borane adduct of cariprazine was formed. By extracting the resulting compound, it has been found that in contrast to most of the Lewis base complexes of borane compounds, this product is not susceptible to moisture in the air, and it can be stored for several years without decomposition. It has been separable from both the starting acid amide and the targeted cariprazine product by thin layer chromatography. The intermediate can be isolated and then converted to cariprazine of formula (1).

For the hydrolysis of the intermediate, a suitable Brönsted acid solution can be used. Brönsted acids include organic and inorganic acids, mainly hydrochloric acid, sulfuric acid, phosphoric acid and acetic acid. The acid solvents used include one-phase and two-phase mixtures of hydrocarbons, ethers, esters and ketones with each other and/or with water, especially mixtures of acetone, methanol, ethanol, isopropanol and t-butanol with water. The intermediate can be thermally decomposed in a commonly used solvent.

The cariprazine salt, preferably a hydrochloride salt prepared according to the process of the present invention can be readily converted by skilled person into a cariprazine base by a simple method known from the state of the art.

The present invention is illustrated by the following non-limiting examples. The structure of the reaction products within the examples was determined by using VNMRS-400 NMR device and by measuring with PANalytical X'Pert PRO MPD X-ray powder diffraction (XRPD) apparatus.

### Examples

### Example 1

### Trans-4-aminocyclohexyl acetic acid

2.21 g of trans-4-aminocyclohexyl acetic acid ethyl ester hydrochloride, 10 ml of methanol and 5 ml of 6N sodium hydroxide solution were charged into a flask. After 2 hours, from the mixture methanol was evaporated in vacuum and then 3 ml solution of 6N hydrochloric acid was dropped to the residue at 0-5 °C temperature. After stirring the suspension for 30 minutes, the product was filtered off and dried under vacuum at 45 °C temperature to constant weight. Thus 0.50 g of product in a white powder form was obtained (yield: 32%; DSC: 259.59-292.07 °C).

¹H NMR (D₂O, 800 MHz): 3.13 (tt, J=12.0, 4.0 Hz, 1H), 2.08 (d, J=7.4 Hz, 2H), 2.03 (br d, J=12.1 Hz, 2H), 1.81 (br d, J=12.9 Hz, 2H), 1.61-1.68 (m, 1H), 1.41 (qua d, J=12.5, 3.3 Hz, 2H), 1..09 (qua d, J=12.6, 3.0 Hz, 2H) ppm. ¹³C NMR (D₂O, 201 MHz): 185.5, 53.0, 47.5, 36.9, 33.1, 32.8 ppm. MS: ESI pos.: [M+H]⁺ = 158; ESI MS/MS, CID=35%, m/z(rel. int. %): 141(100), 123(6).

### Example 2

### Trans-4-aminocyclohexyl-acetic acid hydrochloride

22.2 g of trans-4-aminocyclohexyl-acetic acid ethyl ester hydrochloride and 45 ml of 6N hydrochloric acid solution were charged into a flask. The reaction mixture was refluxed for 16 hours, cooled to 20-25 °C temperature and after 30 minutes stirring the product was filtered off from the resulting suspension, which was dried under vacuum at 45 °C temperature until constant weight. Thus 12.61 g of product is obtained in a white powder form (yield: 65%; DSC: 207.45-211.59°C).

### Example 3

### Trans-4-{[(dimethylamino carbonyl]amino}cyclohexyl} acetic acid

33 ml of water and 1.90 g of trans-4-aminocyclohexyl-acetic acid hydrochloride were charged into a flask, then 3.36 g of sodium bicarbonate was added to the solution. The resulting solution was cooled to 0-5 °C temperature and 1.40 ml dimethyl-carbamoyl chloride was added to that dropwise. The reaction mixture was stirred for 30 minutes at this temperature and then for 2 hours at 20-25 °C. The reaction mixture was cooled again to 0-5 °C and 7.5 ml of 6 N hydrochloric acid was added dropwise. After stirring for 30 minutes, the product was filtered off from the suspension and dried under vacuum at 45 °C temperatures to constant weight. Thus 2.03 g of a product was obtained in a white powder form (yield: 89%; DSC: 206.73-217.37 °C).

¹H NMR (DMSO-*d*₆, 500 MHz): 12.00 (br s, 1H), 5.86 (d, J=7.9 Hz, 1H), 3.32 (tt, J=11.7, 3.8 Hz, 1H), 2.75 (s, 6H), 2.09 (d, J=7.0 Hz, 2H), 1.65-1.78 (m, 4H), 1.50-1.61 (m, 1H), 1.20 (qua d, J=12.8, 2.6 Hz, 2H), 0.98 (qua d, J=12.6, 2.6 Hz, 2H) ppm. MS: El pos.: M⁺ = 228; m/z (rel. int. %): 228(27), 169(14), 156(18), 154(18), 127(28), 124(12), 96(19), 89(41), 88(26), 81(100), 80(39), 72(95), 60(53), 45(61), 44(60).

### Example 4

### 1,1-dimethyl-3-[trans-4-(2-oxo-2-(4-(2,3-dichlorophenyl)piperazin-1-yl-ethyl)cyclohexyl] urea (via acid chloride)

To a round-bottomed flask under nitrogen gas 680 mg of trans-4-{[(dimethylamino)carbonyl]amino}cyclohexyl) acetic acid was dissolved in 3 ml of dichloromethane and to the solution 18 mg dimethylaminopyridine, then 0.7 ml thionyl chloride was added. After three hours, the reaction mixture was evaporated and the evaporation residue was added trough dropping funnel to a suspension of 620 mg of 1-(2,3-dichlorophenyl) piperazine in 4 ml of dichloromethane and 650 mg of sodium bicarbonate under continuous stirring. After the reaction was completed (TCL: DCM / MeOH = 9: 1), 11.0 ml of distilled water was added to the reaction mixture and then it was evaporated to 10.0 g weight under reduced pressure. To the residue 4.0 ml acetone was added, the precipitate was stirred at room temperature for 1 hour, filtered and washed with 0.6 ml of distilled water. It was dried in a vacuum to a constant weight at 45 °C temperature. Thus 0.85 g of product was obtained in a white powder form (yield: 64.4%; DSC: 190.54-195.75 °C).

### Example 5

### 1,1-dimethyl-3-[trans-4-(2-oxo-2-(4-(2,3-dichlorophenyl)piperazin-1-yl-ethyl)cyclohexyl] urea (via acid imidazolidone)

To a round-bottomed flask under nitrogen gas 3.40 g of trans-4-{[(dimethylamino) carbonyl]amino}cyclohexyl)-acetic acid in 20 ml acetone was solved and 2.90 g of carbonyldiimidazole was added to the solution. After 4 hours mixing, 2 ml of isopropanol was added and the mixture was stirred at room temperature for 40 minutes, followed by the addition of 2.1 ml of triethylamine and 3.45 g of 1-(2,3-dichlorophenyl)-piperazine. After stirring for an additional 18 hours at room temperature, 80 ml of distilled water was added. After stirring further for an hour, the precipitated material was filtered off and washed with 2 × 15 ml of distilled water. The product was dried in vacuum at 45 °C temperature to constant weight. Thus 5.37 g of product was obtained in a white powder form (yield: 94.4 %).

¹H NMR (DMSO-*d*₆, 500 MHz): 7.30-7.34 (m, 2H), 7.12-7.17 (m, 1H), 5.85 (d, J=7.9 Hz, 1H), 3.59-3.65 (m, 4H), 3.29-3.39 (m, 1H), 2.89-2.99 (m, 4H), 2.75 (s, 6H), 2.24 (d, J=6.8 Hz, 2H), 1.69-1.78 (m, 4H), 1.58-1.68 (m, 1H), 1.16-1.6 (m, 2H), 0.95-1.05 (m, 2H) ppm. ¹³C NMR (DMSO-*d*₆, 126 MHz): 170.1, 157.6, 150.0, 132.7, 128.5, 126.4, 124.7, 119.9, 51.3, 51.0, 49.2, 45.4, 41.1, 39.1, 35.8, 33.9, 32.7, 31.7 ppm. MS: ESI pos.: [M+H]⁺ = 441; ESI MS/MS, CID=35%, m/z (rel. int. %): 396(23), 353(100), 333(5), 231(3).

### Example 6

### N'-[trans-4-[2-[4-(2,3-dichlorophenyl)-1-piperazinyl]ethyl]cyclohexyl]-N,N-dimethyl urea borane adduct

To a flask used for sulphonation 2.64 g of 1,1-dimethyl-3-[*trans*-4-(2-oxo-2-(4-(2,3-dichlorophenyl)piperazin-1-yl-ethyl)cyclohexyl] urea, 16 ml of tetrahydrofuran and 0.51 g of sodium borohydride were charged, then to the resulting mixture temperature 1.63 ml of boron-trifluoride-diethyl etherate was added at 0-5 °C temperature. At the end of the addition, the mixture was stirred on a temperature between 0°C and 5 °C for an additional hour, then the reaction mixture was warmed to room temperature and 64 ml of distilled water was added. The precipitated crystals were post-mixed for 1 hour, then filtered and washed with 2x4 ml of distilled water. It was dried in vacuum oven to constant weight at 50 ° C temperature. Thus 2.46 g of product was obtained in a white powder form (yield: 92.8%; DSC: 151.51-157.78 °C).

¹H NMR (DMSO-*d*₆, 400 MHz): 7.36-7.30 (m, 2H), 7.15-7.21 (m, 1H), 6.53 (br s, 3H), 5.87 (d, J=7.9 Hz, 1H), 2.60-3.60 (br m, 11H), 2.75 (s, 6H), 1.68-1.81 (m, 4H), 1.44-1.57 (br m, 2H), 1.14-1.28 (m, 3H), 0.90-1.04 (m, 2H) ppm. MS: HRMS (ESI⁺); [M+H]⁺, calcd for C₂₁H₃₆ON₄Cl₂B: 441,23537; found: 441,23551. delta= 0,31 ppm. ESI MS/MS, CID=35%, m/z(rel int %): 427(28), 396(100).

### Example 7

### N'-[trans-4-[2-[4-(2,3-dichlorophenyl)-1-piperazinyl]ethyl]cyclohexyl]-N,N-dimethyl urea

To a round-bottomed flask 100 mg of N'-[trans-4-[2-[4-(2,3-dichlorophenyl)-1-piperazinyl] ethyl]cyclohexyl]-N,N-dimethyl urea borane adduct was charged, then 2.0 ml of tertiary butanol was added and the resulting mixture was heated to reflux. When the reflux temperature was reached, a solution was formed, and crystallization was observed after 30 minutes. TLC was assayed for controlling of completion of the reaction, the suspension was cooled to 0-5 °C temperature and it was stirred for a further hour, then filtered and washed with 2 × 0.2 ml tertiary butanol. The product was dried in vacuum oven at 45 °C temperature until constant weight. The mass of the obtained white crystalline material was 90 mg (yield: 93 %).

¹H NMR (DMSO-*d*₆, 400 MHz): δ 7.18-7.10 (m, 2H), 6.99-6.92 (m, 1H), 4.12 (d, J = 7.5 Hz, 1H), 3.64-3.49 (m, 1H), 3.07 (br s, 4H), 2.88 (s, 6H), 2.63 (br s, 4H), 2.50-2.39 (m, 2H), 2.07-1.94 (m, 2H), 1.82-1.72 (m, 2H), 1.52-1.37 (m, 2H), 1.31-1.18 (m, 1H), 1.18-0.99 (m, 4H). ¹³C NMR δ 157.8 (C), 151.3 (C), 134.0 (C), 127.5 (C), 127.4 (CH), 124.5 (CH), 118.6 (CH), 56.7 (CH₂), 53.4 (CH₂), 51.3 (CH₂), 49.8 (CH), 36.1 (CH₃), 35.7 (CH), 34.0 (CH₂), 33.9 (CH₂), 32.1 (CH₂).

### Example 8

### N'-[trans-4-[2-[4-(2,3-dichlorophenyl)-1-piperazinyl]ethyl]cyclohexyl]-N,N-dimethyl urea borane adduct

To a flask used for sulphonation 1.32 g of 1,1-dimethyl-3-[*trans*-4-(2-oxo-2-(4-(2,3-dichlorophenyl)piperazin-1-yl ethyl)cyclohexyl] urea, 8 ml tetrahydrofuran and 0.23 g sodium borohydride were measured, then, and while we were keeping the temperature between 0 and 5 °C, to the resulting mixture a solution of 8 g of anhydrous aluminum chloride in 4.5 ml tetrahydrofuran was added. At the end of the addition, the mixture was stirred for a further hour at 0-5 °C temperature, then the reaction mixture was warmed to room temperature and after 4 hours of reaction, TLC was assayed for controlling of completion of the reaction. To the mixture 20 ml 2N hydrochloric acid solution was added at 0-5 °C temperature. The precipitated crystals were post-mixed for 1 hour, then filtered and washed with 2 × 2 ml of distilled water. The product was dried in vacuum oven at 50 °C until constant weight. Thus 1.10 g of product was obtained in a white powder form (yield: 83%)

DSC: 110.73 °C decomposition

### Example 9

### N'-[trans-4-[2-[4-(2,3-dichlorophenyl)-1-piperazinyl]ethyl]cyclohexyl]-N,N-dimethyl urea

To a round-bottomed flask 100 mg of N'-[trans-4-[2-[4-(2,3-dichlorophenyl)-1-piperazinyl] ethyl]cyclohexyl]-N,N-dimethyl urea borane adduct was measured, then 2.0 ml of acetone was added and the reaction mixture was heated to reflux. After the reflux was reached, a solution was formed and thin layer chromatography was assayed for completion of the reaction after 24 hours, the suspension was cooled to 0-5 °C temperature, then it was mixed for an additional hour, filtered and washed with 2 × 0.2 ml of acetone. The product was dried in vacuum oven at 45 °C temperature until constant weight. The mass of the obtained white crystalline material was 70 mg (yield: 72 %).

DSC: 201.78 - 209.41°C

### Example 10

### N'-[trans-4-[2-[4-(2,3-dichlorophenyl)-1-piperazinyl]ethyl]cyclohexyl]-N,N-dimethyl urea

To a round-bottomed flask 100 mg of N'-[trans-4-[2-[4-(2,3-dichlorophenyl)-1-piperazinyl]ethyl]cyclohexyl]-N,N-dimethyl urea borane adduct was measured, then 2.0 ml methyl isobutyl ketone was added and the reaction mixture was heated to reflux. After the reflux was reached, a solution was formed and after 15 minutes a thin layer chromatograph was assayed for completion of the reaction, the suspension was cooled to 0-5 °C temperature, then it was mixed further for one hour, filtered and washed with 2 × 0.2 ml of methyl isobutyl ketone. The product was dried in vacuum oven at 45 °C temperature until constant weight. The mass of the obtained white crystalline material was 70 mg (yield: 72 %).

DSC: 200.91 - 208.88°C

### Example 11

### N'-[trans-4-[2-[4-(2,3-dichlorophenyl)-1-piperazinyl] ethyl] cyclohexyl]-N,N-dimethyl urea

To a round-bottomed flask 100 mg of N'-[trans-4-[2-[4-(2,3-dichlorophenyl)-1-piperazinyl] ethyl]cyclohexyl]-N, N-dimethyl urea borane adduct was measured, then a mixture of 1.9 ml dimethylacetamide and 0.1 ml of water was added. The suspension was heated to 45 °C temperature. Within one hour, the product was precipitated from the resulting solution. The suspension was cooled to 0-5 °C temperature, then it was mixed further for one hour, it was filtered and washed with 2 × 0.2 ml of water. The product was dried in vacuum oven at 45 °C temperature until constant weight. The mass of the obtained white crystalline material was 90 mg (yield: 93 %).

DSC: 202.70 - 210.60 °C

### Example 12

### N'-[trans-4-[2-[4-(2,3-dichlorophenyl}-1-piperazinyl]ethyl]cyclohexyl]-N,N-dimethyl urea dihydrochloride

To a round-bottomed flask 200 mg of N'-[trans-4-[2-[4-(2,3-dichlorophenyl)-1-piperazinyl] ethyl]cyclohexyl]-N,N-dimethyl urea borane adduct was measured, then 1.0 ml 6N hydrochloride solution was added and the mixture was heated to reflux. After the reflux was reached, 2.0 ml of distilled water was added, and the reaction mixture was cooled to 0-5 °C temperature, then it was mixed further for one hour, it was filtered and washed with 2 × 0.5 ml of water. The product was dried in vacuum oven at 45 °C temperature until constant weight. The mass of the obtained white crystalline material was 180 mg (yield: 79.4 %). XRPD peaks °2θ (% Rel. Int.): 7.2 (8.7); 11.1 (14.4); 13.0 (40.2); 13.8 (47.0); 14.0 (30.1); 14.4 (41.4); 15.0 (86.3); 18.4 (100.0); 22.3 (37.9); 24.6 (70.5); 25.3 (58.6).

### Example 13

### N'-[trans-4-[2-[4-(2,3-dichlorophenyl)-1-piperazinyl]ethyl]cyclohexyl]-N,N-dimethyl urea hydrochloride

To a round-bottomed flask 200 mg of N'- [trans-4-[2-[4-(2,3-dichlorophenyl)-1-piperazinyl]ethyl-cyclohexyl]-N,N-dimethyl urea dihydrochloride, 0.8 ml of methanol and 0.2 ml of 2N hydrochloric acid were measured. To the resulting solution 3.2 ml of distilled water was added dropwise over 20 minutes. The resulting slurry was stirred at 20-25 °C, then it was filtered and washed with 2×50 ml of distilled water. The product was dried in vacuum oven at 45 °C temperature until constant weight. The mass of the obtained white crystalline material was 150 mg (yield: 81.0 %).

XRPD peaks °2θ (% Rel. Int.): 6.6 (4.9); 7.3 (50.0); 13.2 (53.1); 14.3 (100.0); 14.6 (56.7); 16.9 (89.4); 21.1 (72.9); 22.4 (95.6); 24.8 (51.8); 26.5 (75.6); 26.8 (19.6).

### Example 14

### N'-[trans-4-[2-[4-(2,3-diehlorophenyl)-1-piperazinyl]ethyl]cyclohexyl]-N,N-dimethyl urea hydrochloride

To a round-bottomed flask 400 mg of N'-[trans-4-[2-[4-(2,3-dichlorophenyl)-1-piperazinyl] ethyl]cyclohexyl]-N,N-dimethyl urea borane adduct was measured, then 0.3 ml of 6N hydrochloric acid, 0.1 ml of water and 1.6 ml of methanol were added. The reaction mixture was stirred at 60-65 °C temperature for 3 hours. The completion of the chemical reaction was checked, the reaction mixture was cooled and the methanol was evaporated in vacuum. The residue was taken up in 1.6 ml of distilled water, then it was stirred for 1 hour at room temperature and at 0-5 °C temperature for an additional hour, it was filtered and washed with 2×50 ml of distilled water.

The product was dried in vacuum oven at 45 °C temperature until constant weight. The mass of the obtained white crystalline material was 397 mg (yield: 94.4 %).

XRPD peaks °2θ (% Rel. Int.): 6.6 (5.3); 7.3 (44.0); 13.1 (50.3); 14.2 (84.6); 14.6 (50.4); 16.9 (88.9); 21.1 (71.5); 22.4 (100.0); 24.8 (53.1); 26.5 (62.5); 26.8 (14.3).

### Example 15

### N'-[trans-4-[2-[4-(2,3-dichlorophenyl)-1-piperazinyl]ethyl]cyclohexyl]-N,N-dimethyl urea

To a round-bottomed flask 1.00 g N'-[trans-4-[2-[4-(2,3-dichlorophenyl)-1-piperazinyl]ethyl]cyclohexyl]-N,N-dimethyl urea hydrochloride was measured, then 10 ml dichloromethane and 5 ml of saturated sodium bicarbonate solution was added. After stirring the reaction mixture for 15 minutes, the phases were separated and the aqueous phase was washed with 5 ml dichloromethane. The combined organic phase was dried and concentrated in vacuum. The 0.89 g of the evaporation residue was stirred in 6 ml of isopropyl alcohol for 15 minutes, filtered and washed with 2 × 1 ml of isopropyl alcohol. The product was dried in vacuum oven at 45 °C temperature until constant weight. The mass of the obtained white crystalline material was 0.83 g (yield: 90.0 %).

DSC: 209.58 - 213.72°C

## Claims

1. Process for the preparation of cariprazine or a salt thereof 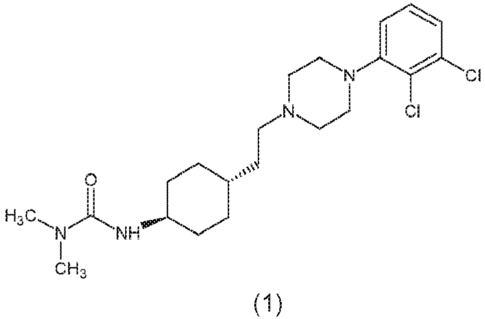 wherein
a) (trans-4-amino-cyclohexyl)-acetic acid ethyl ester hydrochloride is converted to trans-4-aminocyclohexyl) acetic acid or its hydrochloride by hydrolysis,
b) from the obtained product with addition of a dicarbamoyl derivative selected from a halide, imidazolide, anhydride or active ester as a suitable reagent in presence of alkaline reagent (trans-4-{[(dimethylamino)carbonyl]amino} cyclohexyl) acetic acid is formed,
c) then the obtained compound is linked to 1-(2,3-dichlorophenyl)-piperazine in the presence of carboxylic acid activating coupling reagent, and so 1,1-dimethyl-3-[trans-4-(2-oxo-2-(4-(2,3-dichlorophenyl)piperazin-1-yl-ethyl)cyclohexyl] urea is formed,
d) which is converted to cariprazine borane adduct of formula (2), 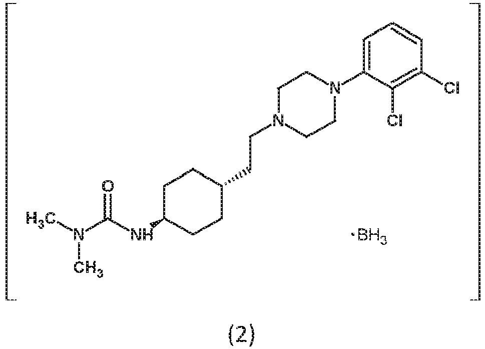 in the presence of a borohydride compound, borane or a borane complex as a reducing agent,
e) and the final product itself is eliminated directly or is obtained from its salt.

2. Process according to claim 1 **characterized by** carrying out the hydrolysis of (trans-4-amino--cyclohexyl)--acetic acid ethyl ester hydrochloride by basic or acidic hydrolysis.

3. Process according to claims 1 or 2 **characterized in that** (trans-4-{[(dimethylamino)carbonyl]amino}cyclohexyl)-acetic acid is formed with dimethyl-carbamoyl chloride as reagent.

4. Process according to any of the claims 1 to 3 **characterized in that** the carboxylic acid activating coupling reagent for coupling of 1-(2,3-dichlorophenyl)-piperazine is a dehydrating agent.

5. Process according to any of the claims 1 to 3 **characterized in that** for coupling reaction with 1-(2,3-dichlorophenyl)-piperazine carbonyldiimidazole or thionyl chloride is used.

6. Process according to any of the claims 1 to 5 **characterized in that** the reducing agent is borane which is formed "in situ" from a suitable precursor.

7. Process according to any of the claims 1 to 6 **characterized in that** borohydrides or boranes or complexes thereof having the corresponding counter ion are used as reducing agent.

8. Process according to claim 7 **characterized in that** borane complex refers to the complexes of boranes formed with Lewis bases, such as complexes formed with ethers, thioethers, amines, preferably a complex formed with tetrahydrofuran.

9. Process according to claim 7 **characterized in that** suitable counter ion for borohydrides are positive ions of metallic elements such as alkali metals, alkaline earth metals, earth metals, preferably aluminum.

10. Process according to any of the claims 1 to 9 **characterized in that** after isolation of cariprazine borane adduct, carprazine salts are obtained by decomposition with Brönsted acids in conventional solvents.

11. A process according to claim 10 **characterized in that** after isolation of cariprazine borane adduct, Brönsted acid used for decomposition in conventional solvents is hydrogen chloride.

12. Process according to any of the claims 1 to 11 **characterized in that** the resulting cariprazine hydrochloride salts are converted into base final product.

13. Process according to any of the claims 1 to 9 **characterized in that** from the isolated cariprazine borane adduct in conventional solvents by thermal decomposition carprazine base final product is obtained. urea

## Patentansprüche

1. Verfahren zur Herstellung von Cariprazin oder einem Salz davon 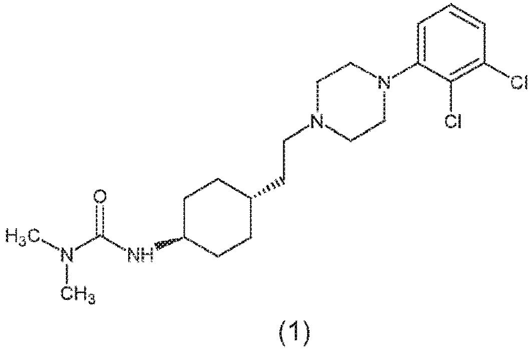 worin
a) (trans-4-Aminocyclohexyl)essigsäureethylester-hydrochlorid durch Hydrolyse in (trans-4-Aminocyclohexyl)essigsaure oder ein Hydrochlorid davon umgewandelt wird,
b) aus dem erhaltenen Produkt unter Zugabe eines Dicarbamoylderivats, ausgewählt aus Halogenid, Imidazolid, Anhydrid und Aktivester, als geeignetes Reagenz in Gegenwart eines alkalischen Reagenzes (trans-4-{[(Dimethylamino)carbonyl]amino}cyclohexyl)essigsäure gebildet wird,
c) dann die erhaltene Verbindung mit 1-(2,3-Dichlorphenyl)piperazin in Gegenwart eines carbonsäureaktivierenden Kupplungsmittels verbunden wird, und so 1,1-Dimethyl-3-[trans-4-(2-oxo-2-(4-(2,3-dichlorphenyl)piperazin-1-yl-ethyl)-cyclohexyl]harnstoff gebildet wird,
d) welcher in ein Cariprazin-Boran-Addukt der Formel (2) 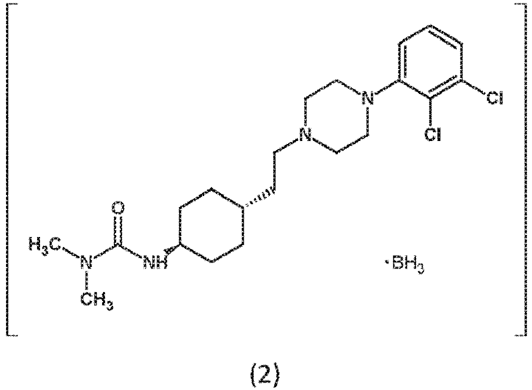 in Gegenwart einer Borhydridverbindung, Borans oder eines Borankomplexes als Reduktionsmittel umgewandelt wird,
e) und das Endprodukt selbst direkt eliminiert wird oder aus seinem Salz erhalten wird.

2. Verfahren gemäß Anspruch 1, **dadurch gekennzeichnet, dass** die Hydrolyse von (trans-4-Aminocyclohexyl)essigsäure-ethylesterhydrochlorid mittels basischer oder saurer Hydrolyse durchführt wird.

3. Verfahren gemäß Anspruch 1 oder 2, **dadurch gekennzeichnet, dass** (trans-4-{[(Dimethylamino)carbonyl]amino}cyclohexyl) essigsäure mit Dimethylcarbamoylchlorid als Reagenz gebildet wird.

4. Verfahren gemäß einem der Ansprüche 1 bis 3, **dadurch gekennzeichnet, dass** das carbonsäureaktivierende Kupplungsmittel zur Kupplung von 1-(2,3-Dichlorphenyl)piperazin ein Dehydratisierungsmittel ist.

5. Verfahren gemäß einem der Ansprüche 1 bis 3, **dadurch gekennzeichnet, dass** für die Kupplungsreaktion mit 1-(2,3-Dichlorphenyl)piperazin Carbonyldiimidazol oder Thionylchlorid verwendet wird.

6. Verfahren gemäß einem der Ansprüche 1 bis 5, **dadurch gekennzeichnet, dass** das Reduktionsmittel Boran ist, das "in situ" aus einem geeigneten Vorläufer gebildet wird.

7. Verfahren gemäß einem der Ansprüche 1 bis 6, **dadurch gekennzeichnet, dass** Borhydride oder Borane oder Komplexe davon mit dem entsprechenden Gegenion als Reduktionsmittel verwendet werden.

8. Verfahren gemäß Anspruch 7, **dadurch gekennzeichnet, dass** sich der Boran-Komplex auf die mit Lewis-Basen gebildeten Komplexe von Boranen, wie zum Beispiel mit Ethern, Thioethern, Aminen gebildete Komplexe, vorzugsweise einen mit Tetrahydrofuran gebildeten Komplex, bezieht.

9. Verfahren gemäß Anspruch 7, **dadurch gekennzeichnet, dass** geeignete Gegenionen für Borhydride positive Ionen metallischer Elemente, wie zum Beispiel Alkalimetalle, Erdalkalimetalle, Erdmetalle, vorzugsweise Aluminium, sind.

10. Verfahren gemäß einem der Ansprüche 1 bis 9, **dadurch gekennzeichnet, dass** nach Isolierung des Cariprazin-Boran-Addukts Cariprazin-Salze durch Zersetzen mit Brönsted-Säuren in üblichen Lösungsmitteln erhalten werden.

11. Verfahren gemäß Anspruch 10, **dadurch gekennzeichnet, dass** nach der Isolierung des Cariprazin-Boran-Addukts die zur Zersetzung in herkömmlichen Lösungsmitteln verwendete Brönsted-Säure Chlorwasserstoff ist.

12. Verfahren gemäß einem der Ansprüche 1 bis 11, **dadurch gekennzeichnet, dass** die erhaltenen Cariprazin-Hydrochloridsalze in das Basen-Endprodukt überführt werden.

13. Verfahren gemäß einem der Ansprüche 1 bis 9, **dadurch gekennzeichnet, dass** aus dem isolierten Cariprazin-Boran-Addukt durch thermisches Zersetzen in herkömmlichen Lösungsmitteln ein Cariprazinbase-Endprodukt erhalten wird.

## Revendications

1. Procédé de préparation de cariprazine ou un sel de celle-ci 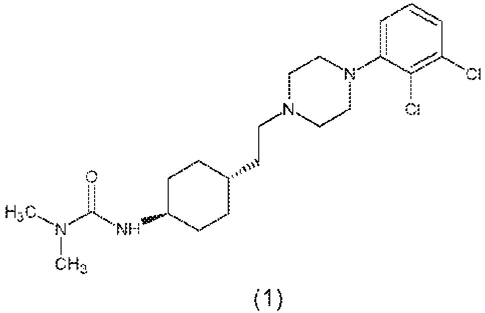 dans lequel
a) du chlorhydrate d'ester éthylique d'acide (trans-4-amino-cyclohexyl)-acétique est converti en acide trans-4-aminocyclohexyl) acétique ou en son chlorhydrate par hydrolyse,
b) à partir du produit obtenu avec ajout d'un dérivé de dicarbamoyle sélectionné parmi un halogénure, un imidazolide, un anhydride ou un ester actif en tant que réactif approprié en présence d'un réactif alcalin, de l'acide (trans-4-{[(diméthylamino)carbonyl]amino} cyclohexyl) acétique est formé,
c) puis le composé obtenu est lié à de la 1-(2,3-dichlorophényl)-pipérazine en présence d'un agent de couplage activateur d'acide carboxylique, et ainsi de la 1,1-diméthyl-3-[trans-4-(2-oxo-2-(4-(2,3-dichlorophényl)pipérazin-1-yl-éthyl)cyclohexyl] urée est formée,
d) qui est convertie en adduit cariprazine borane de formule (2), 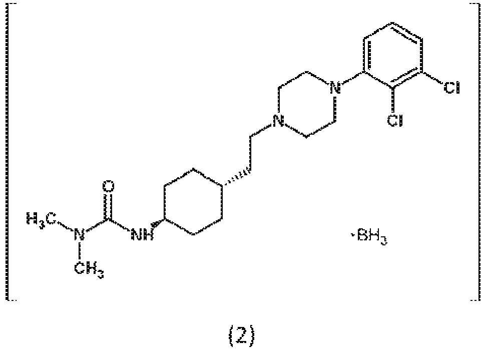 en présence d'un composé borohydrure, de borane ou d'un complexe borane en tant qu'agent réducteur,
e) et le produit final lui-même est directement éliminé ou est obtenu à partir de son sel.

2. Procédé selon la revendication 1, **caractérisé par** la réalisation de l'hydrolyse du chlorhydrate d'ester éthylique d'acide (trans-4-amino-cyclohexyl)-acétique par hydrolyse basique ou acide.

3. Procédé selon les revendications 1 ou 2, **caractérisé en ce que** l'acide (trans-4-{[(diméthylamino}carbonyl]amino}cyclohexyl}-acétique est formé avec du chlorure de diméthyl-carbamoyle en tant que réactif.

4. Procédé selon l'une quelconque des revendications 1 à 3, **caractérisé en ce que** le réactif de couplage activateur d'acide carboxylique pour le couplage de la 1-(2,3-dichlorophényl)-pipérazine est un agent déshydrateur.

5. Procédé selon l'une quelconque des revendications 1 à 3, **caractérisé en ce que** pour la réaction de couplage avec de la 1-(2,3-dichlorophényl)-pipérazine, du carbonyldiimidazole ou du chlorure de thionyle est utilisé.

6. Procédé selon l'une quelconque des revendications 1 à 5, **caractérisé en ce que** l'agent réducteur est le borane qui est formé « in situ » à partir d'un précurseur approprié.

7. Procédé selon l'une quelconque des revendications 1 à 6, **caractérisé en ce que** des borohydrures ou des boranes ou des complexes de ceux-ci présentant le contre-ion correspondant sont utilisés en tant qu'agent réducteur.

8. Procédé selon la revendication 7, **caractérisé en ce que** le complexe borane fait référence aux complexes de boranes formés avec des bases de Lewis, tels que des complexes formés avec des éthers, des tioéthers, des amines, de préférence un complexe formé avec du tétrahydrofuranne.

9. Procédé selon la revendication 7, **caractérisé en ce que** le contre-ion approprié pour les borohydrures sont des ions positifs d'éléments métalliques tels que des métaux alcalins, des métaux alcalino-terreux, des métaux terreux, de préférence l'aluminium.

10. Procédé selon l'une quelconque des revendications 1 à 9, **caractérisé en ce qu'**après l'isolement de l'adduit cariprazine borane, des sels de carprazine sont obtenus par décomposition avec des acides
de Brönsted dans des solvants classiques.

11. Procédé selon la revendication 10, **caractérisé en ce qu'**après l'isolement de l'adduit cariprazine borane, l'acide de Brönsted utilisé pour la décomposition dans des solvants classiques est le chlorure d'hydrogène.

12. Procédé selon l'une quelconque des revendications 1 à 11, **caractérisé en ce que** les sels de chlorydrate de cariprazine résultants sont convertis en un produit final de base.

13. Procédé selon l'une quelconque des revendications 1 à 9, caractérisé en qu'à partir de l'adduit cariprazine borane isolé dans des solvants classiques par décomposition thermique, un produit final de base de carprazine est obtenu.
